# EUROPEAN PATENT SPECIFICATION

(11) **EP 3 238 725 B1**
(45) Date of publication and mention of the grant of the patent: **02.02.2022**
(21) Application number: 15872795.8
(22) Date of filing: 14.12.2015
(51) Int. Cl.: A61K 31/618, A61K 9/70, A61K 47/10, A61K 47/14, A61K 47/24, A61K 47/32

(54) **POULTICE**
UMSCHLAG
CATAPLASME

(30) Priority: 22.12.2014 JP 2014258878
(43) Date of publication of application: 01.11.2017
(73) Proprietor: Hisamitsu Pharmaceutical Co., Inc., Tosu-shi, Saga 841-0017 (JP)
(72) Inventor: TSURUSHIMA Keiichiro, Tosu-shi Saga 841-0017 (JP); KOSE Yasuhisa, Tosu-shi Saga 841-0017 (JP); YOSHINAGA Takaaki, Tosu-shi Saga 841-0017 (JP)
(74) Representative: Grünecker Patent- und Rechtsanwälte PartG mbB
(86) International application number: PCT/JP2015/084970
(87) International publication number: WO 2016/104226

(56) References cited:
- WO-A1-02/100384
- WO-A1-03/082164
- WO-A1-2006/090782
- WO-A1-2009/125667
- JP-A- S61 200 918
- JP-A- 2005 126 341
- None

## Description

### Technical Field

The present invention relates to a gel patch.

### Background Art

Gel patches are one type of patches produced by applying a medicament-containing adhesive mass on a backing fabric, and generally contains a large amount of moisture, and the adhesive mass is thick. Since the gel patch has such a configuration, the permeation of active ingredients into the skin is promoted to reduce skin irritation.

### Citation List

### Patent Literature

[Patent Literature 1] JP H10-114647 A
WO 02/100384 and WO 03/082164 relate to a sheet-like patch agent; JP 2005-126341 discloses a water-containing antiinflammatory analgesic plaster; WO 2009/1256667 relates to a butenafine hydrochloride-containing aqueous patch; WO 2006/090782 discloses a composition for a water-containing external patch; JP S61-200918 discloses an antiinflammatory analgesic agent for external use.

### Summary of Invention

### Technical Problem

In a gel patch containing glycol salicylate, however, the storage stability of glycol salicylate in the adhesive mass layer is low, and in some cases, it is not possible to exhibit analgesic action sufficiently after the storage for a long period of time.

Therefore, it is an object of the present invention to provide a gel patch with an excellent storage stability of glycol salicylate.

### Solution to Problem

The present invention provides a gel patch comprising an adhesive mass layer on a backing fabric, wherein the adhesive mass layer comprises glycol salicylate, a water-soluble polymer, water and glycerin and poly(methyl acrylate/2-ethylhexyl acrylate), and the content of glycerin is 8 to 15 times the content of glycol salicylate on the basis of mass, wherein the pH of the adhesive mass layer is 4.7 to 5.1.

With necessarily involving the above-described ingredients, a gel patch of the present invention has such a constitution that the content ratio between glycol salicylate and glycerin becomes a particular proportion. On the basis of such a constitution, the storage stability of glycol salicylate remarkably improves.

Note that, by setting the content of glycerin to be 8 to 14 times the content of glycol salicylate on the basis of mass, it is possible to further enhance the storage stability of glycol salicylate.

It is preferable that the pH of the above-described adhesive mass layer be 4.9 to 5.1. With the pH of 4.7 or more (further 4.9 or more), the cohesive strength of the adhesive mass tends to become better, and with the pH of 5.1 or less, the storage stability of glycol salicylate tends to become better.

Also, the above-described adhesive mass layer comprises poly(methyl acrylate/2-ethylhexyl acrylate). By incorporation of poly(methyl acrylate/2-ethylhexyl acrylate) into the adhesive mass layer, it is possible to maintain the adhesion force after the elapse of a long period of time.

It is preferable that the above-described water-soluble polymer contain a polyacrylic acid or a neutralized polyacrylate. By incorporation of these ingredients, it is possible to improve the adhesion property with an enhanced storage stability of glycol salicylate.

### Advantageous Effects of Invention

The gel patch according to the present invention provides an excellent storage stability of glycol salicylate, and can exhibit a sufficient analgesic action even after the storage for a long period of time. Also, the moisture retention required for the gel patch is ensured.

### Description of Embodiments

Hereinbelow, the present invention is illustrated by showing one embodiment of the present invention.

In the present specification, the storage stability of glycol salicylate means that the content of glycol salicylate after the storage at 60°C for one month is 89% or more in relation to that at the time of preparation of the gel patch.

One embodiment of the present invention is a gel patch comprising an adhesive mass layer on a backing fabric, wherein the adhesive mass layer contains glycol salicylate, a water-soluble polymer, water and glycerin, and poly(methyl acrylate/2-ethylhexyl acrylate), and the content of glycerin is 8 to 15 times the content of glycol salicylate on the basis of mass, and the pH of the adhesive mass layer is 4.7 to 5.1.

Examples of the backing fabric include woven fabrics, nonwoven fabrics, resin films, foamed sheets and paper, and examples of the woven fabric include knitted fabrics. In the case of using a woven fabric, nonwoven fabric or resin film as a backing fabric, examples of the fabric include polyolefins such as polyethylene, polypropylene and polybutylene, polyesters such as polyethylene terephthalate, rayon, polyurethanes and cotton, and these can be used singly or in combination of two or more. Also, the backing fabric can have a monolayer structure, or can have a multilayered structure. As the fabric of the backing fabric, polyesters are more preferable.

As a backing fabric, a nonwoven fabric or woven fabric is preferable, and a nonwoven fabric or woven fabric having a certain elongation recovery rate is particularly preferable. Here, an elongation recovery rate is a value measured according to "JIS L 1096 Testing Methods for Woven and Knitted Fabrics". It is preferable to use a nonwoven fabric or woven fabric having an elongation recovery rate because, when the fabrics are attached to a movable body part such as the joint, the backing fabric stretches according to the movement of the part to which the fabric is attached.

In the case where the backing fabric is a nonwoven fabric, it is preferable that a load at 50% elongation be, for example, 1 to 5 N/2.5 cm in the longitudinal direction (long axis direction) and 0.1 to 3 N/2.5 cm in the transverse direction (short axis direction). Also, the 50% elongation recovery rate is, for example, 60 to 99%, and preferably 65 to 95%, and more preferably 70 to 90%. A suitable mass per unit area of the backing fabric is, for example, 80 to 120 g/m², and is preferably 90 to 110 g/m². A suitable thickness of the backing fabric is, for example, 0.5 to 2 mm. Also, a bending resistance of the backing fabric (the measurement method of bending resistance is in accordance with JIS L 1096 45° cantilever method.) can be, for example, 20 to 40 mm in the longitudinal direction (long axis direction) and 10 to 35 mm in the transverse direction (short axis direction), and is preferably 25 to 35 mm in the longitudinal direction (long axis direction), and 15 to 30 mm in the transverse direction (short axis direction).

In the case where a woven fabric, particularly a knitted fabric is used as the backing fabric, examples of the woven fabric include a knitted fabric obtained by assembling the stitches such as by circular knitting, warp knitting or weft knitting for processing into the form of a cloth. Preferred examples of the knitted fabric include knitted fabrics prepared by using fabrics such as polyester-based fabric, nylon-based fabric, polypropylene-based fabric or rayon-based fabric singly or in combination of two or more, and among these, a knitted fabric consisting of a polyester-based polyethylene terephthalate having a little interaction with drugs is more preferable.

In particular, in the case where the backing fabric is a woven fabric, it is preferable that the load at 50% elongation be, for example, 1 to 5 N/2.5 cm in the longitudinal direction (long axis direction) and 0.1 to 3 N/2.5 cm in the transverse direction (short axis direction). Also, the 50% elongation recovery rate is, for example, 60 to 99%, and preferably 65 to 95%, and more preferably 70 to 90%. Also, the bending resistance of the backing fabric can be, for example, 10 to 30 mm in the longitudinal direction (long axis direction), and 10 to 30 mm in the transverse direction (short axis direction), and is preferably 15 to 25 mm in the longitudinal direction (long axis direction), and 15 to 25 mm in the transverse direction (short axis direction).

When an adhesive mass containing water is spread over a woven fabric, water can seep through the mesh of the woven fabric, and by setting the mass per unit area of the polyethylene terephthalate woven fabric to be 80 to 150 g/m², there is a tendency to ensure the ability for water contained in the adhesive mass to be spread over without the seeping through the mesh of the woven fabric, as well as to enable to maintain the anchoring between the woven fabric and the adhesive mass.

Also, it is preferable that the polyethylene terephthalate woven fabric have a modulus of 2 to 12 N/5 cm in the longitudinal direction (long axis direction) and a modulus of 2 to 8 N/5 cm in the transverse direction (short axis direction) (The measurement method of the modulus is in accordance with JIS L 1018.). With a modulus lower than 2 N/5 cm (longitudinal direction) or 2 N/5 cm (transverse direction), when the adhesive mass is applied, the woven fabric stretches so that the pressure-sensitive adhesive may penetrate into the mesh to diminish the performance as a gel patch. Also, with a modulus higher than 12 N/5 cm (longitudinal direction) or 8 N/5 cm (transverse direction), the stretchability becomes worse, and when the adhesive mass is applied to the bending portion, the adhesive mass may be sometimes difficult to follow the stretching of the skin.

Glycol salicylate contained in the adhesive mass layer is one of non-steroidal anti-inflammatory analgesics and has an effect to alleviate inflammatory symptoms. Also, glycol salicylate is a compound having an anti-inflammatory analgesic action and has a higher water solubility than methyl salicylate.

The adhesive mass layer contains a water-soluble polymer, and this water-soluble polymer means a polymer having a hydrophilic group. Examples of the hydrophilic group include a hydroxy group, a carboxy group, and an amino group. By the incorporation of the water-soluble polymer, it is possible to retain the moisture in the gel patch for a longer period of time.

It is preferable that the water-soluble polymer contain a polyacrylic acid or a neutralized polyacrylate (In some cases, these are referred to as "water-soluble acrylic polymer".). Due to the fact that the adhesive mass layer contains a polyacrylic acid or its neutralization product, it is possible to obtain a gel patch with an excellent adhesion.

In the case where a polyacrylic acid is contained in the adhesive mass layer as a water-soluble polymer, it is preferable that the content of the polyacrylic acid be 1 to 5% by mass on the basis of the total mass of the adhesive mass layer, and it is more preferable that the content be 2 to 6% by mass. By setting the content of the water-soluble polymer to be 1% by mass or more, the moldability and shape retention ability of the adhesive mass layer tend to improve, and, by setting the content of the polyacrylic acid to be 5% by mass or less, the hardness of the adhesive mass layer tends not to become high, so that the adhesion to skin will become higher.

The neutralized polyacrylate can be a polyacrylic-acid full-neutralization product, a polyacrylic-acid partial-neutralization product, or a mixture thereof. Examples of the neutralized polyacrylate include polyacrylic acid salts, and for example, it is possible to use a sodium salt, a potassium salt, a calcium salt, an ammonium salt or the like.

As the neutralized polyacrylate, partially neutralized polyacrylate is preferable because the initial adhesion force and the overtime sustainability of the adhesion force become higher. The partially neutralized polyacrylate is a partial-neutralization product in which structural units derived from an acrylic acid and structural units derived from an acrylic acid salt are present in one polymer chain in an arbitrary proportions. As a partially neutralized polyacrylate, it is preferable to use a partially neutralized polyacrylate in which 20 to 80 mol% of the carboxy groups in one polymer chain have been neutralized.

In the case where a neutralized polyacrylate is contained in the adhesive mass layer as a water-soluble polymer, it is preferable that the content of the neutralized polyacrylate be 1 to 6% by mass on the basis of the total mass of the adhesive mass layer, and it is more preferable that the content be 2 to 6% by mass. By setting the content of the neutralized polyacrylate to be 1% by mass or more, the adhesion force of the neutralized polyacrylate can be sufficiently obtained, and by setting the content of the neutralized polyacrylate to be 6% by mass or less, the moldability and shape retention ability of the adhesive mass layer improve. Note that, in the case of using a polyacrylic acid and a neutralized polyacrylate (preferably, partially neutralized polyacrylate) in combination as water-soluble polymers, the suitable contents of each of these are as described above.

As the water-soluble polymer, polymers other than the water-soluble acrylic polymers, for example, gelatin, polyvinyl alcohol, polyvinyl pyrrolidone, sodium alginate, hydroxypropyl cellulose, carboxymethyl cellulose sodium (carmellose sodium), methyl cellulose, and carrageenan can be used. These can be used singly, or used in combination of two or more. It is preferable that a water-soluble polymer is carmellose sodium, gelatin or polyvinyl alcohol. Note that these can be used in combination with a water-soluble acrylic polymer.

In the case where a water-soluble polymer other than the water-soluble acrylic polymers is contained in the adhesive mass layer as the water-soluble polymer, it is preferable that the content of the water-soluble polymer be 3 to 10% by mass on the basis of the mass of the adhesive mass layer. When the content of the water-soluble polymer is 3% by mass or more, the cohesive strength of the adhesive mass layer tends to be high, and when the content of the water-soluble polymer is 10% by mass or less, glycol salicylate contained in the adhesive mass layer tends to disperse homogenously.

Glycerin exhibits an effect to suppress the evaporation of moisture from the adhesive mass layer of the gel patch. It is preferable for the content of glycerin to be 3 to 70% by mass on the basis of the mass of the adhesive mass layer, and it is more preferable to be 4 to 60% by mass.

Also, it is preferable for the content of glycerin to be 8 to 14 times the content of glycol salicylate on the basis of mass, and it is more preferable to be 8 to 13.6 times.

By incorporation of water into the adhesive mass layer, the skin permeability of glycol salicylate improves not only to alleviate heat sensation, which is one of inflammatory symptoms, but also to exhibit the anti-inflammatory analgesic action of glycol salicylate more effectively.

It is preferable for the content of water to be 10 to 90% by mass on the basis of the mass of the adhesive mass layer, and it is more preferable to be 15 to 88% by mass, and it is further preferable to be 18 to 85% by mass.

The mass of the adhesive mass layer can be, for example, 214 to 1000 g/m², 400 to 1000 g/m², or 400 to 650 g/m². Preferably, by setting the mass of the adhesive mass layer to be 400 to 650 g/m², the adhesion for a longer period can be improved with an excellent feeling of fit. In the case where the mass of the adhesive mass layer is in the above-described range, it is possible to reduce the thickness of the entire gel patch, and the gel patch easily follow the skin, and in addition, when the gel patch is attached to the skin, the step height in relation to the peripheral portions become smaller, and therefore, the gel patch tends not to peel easily.

The adhesive mass layer further contains poly(methyl acrylate/2-ethylhexyl acrylate). In the case of conventional gel patches, when the adhesive mass layer has a small weight, the water content is likely to lower, and the adhesion force is likely to lower. However, since the adhesive mass layer contains poly(methyl acrylate/2-ethylhexyl acrylate), even in the case where the mass of the adhesive mass layer is relatively small, a sufficient adhesion force tends to be maintained after a long period of time has elapsed

As the poly(methyl acrylate/2-ethylhexyl acrylate), an aqueous emulsion using water as a medium is preferable. Also, it is preferable that the poly(methyl acrylate/2-ethylhexyl acrylate) emulsion be an emulsion using polyoxyethylene nonyl phenyl ether as a surfactant or protective colloid. Also, it is preferable that the evaporation residue (nonvolatile content) by heating at the boiling point of the medium or higher (for example, at 105°C for 3 hours) be 57 to 61%. As such an emulsion, NIKASOL TS-620 (trade name, manufactured by NIPPON CARBIDE INDUSTRIES CO., INC.) is mentioned. According to Japanese Pharmaceutical Excipients (2013), after the evaporation of NIKASOL TS-620 to dryness over a water bath followed by drying at 105°C for 3 hours, the amount of the evaporation residue is 57 to 61%.

It is preferable that the pH of the adhesive mass layer be 4.9 to 5.1. By setting the pH to be 4.7 or more, skin irritation decreases, and by setting the pH to be 5.1 or less, it is possible to improve the moldability and shape retention ability of the gel patch. In particular, in the case where the backing fabric is a woven fabric, particularly knitted fabric, the seeping can occur during the formation of the adhesive mass layer, and in the case where the pH is 4.9 to 5.1, the seeping tends to be suppressed. Note that the pH can be, for example, measured using glass-combined electrodes by referring to the pH measurement method of Japanese Pharmacopoeia General Test Method, by subjecting the sample to 20 fold dilution with purified water.

As other ingredients it is also possible to further add other medicaments, dissolution aid, crosslinker, humectant, cooling agent, stabilizer, inorganic powder, colorant, flavoring agent, pH modifier or the like to the adhesive mass layer.

Other medicaments can be any medicament having percutaneous absorption ability, and examples of the other medicaments include non-steroidal anti-inflammatory drugs such as felbinac, flurbiprofen, diclofenac, diclofenac sodium, methyl salicylate, indometacin, ketoprofen, ibuprofen or esters thereof, anti-histamine drugs such as diphenhydramine or chlorpheniramine, analgesics such as aspirin, acetaminophen, ibuprofen, or loxoprofen sodium, local anesthetics such as lidocaine or dibucaine, muscle relaxants such as suxamethonium chloride, antifungal agents such as clotrimazole, antihypertensive drugs such as clonidine, vasodilators such as nitroglycerin or isosorbide nitrate, vitamins such as vitamin A, vitamin E (tocopherol), tocopherol acetate, vitamin K, octotiamine, riboflavin butyrate, prostaglandins, scopolamine, fentanyl, Capsicum extract, vanillylamide nonylate.

Also, the adhesive mass layer may contain an ingredient derived from fruits such as rose fruit extract, orange extract, orange fruit juice, raspberry extract, kiwi extract, cucumber extract, Gardenia extract, grapefruit extract, Crataegus Cuneata extract, Japanese pepper extract, Crataegus Oxyacantha extract, Juniperus Communis extract, jujube extract, Lansium Domesticum extract, tomato extract, grape extract, Luffa Cylindrica extract, lime fruit juice, apple extract, apple fruit juice, lemon extract, or lemon fruit juice, an ingredient extracted from various galenicals such as water-soluble placenta extract, allantoin, lecithin, amino acids, kojic acid, proteins, saccharides, hormones, placenta extract, or aloe and licorice, Angelica Keiskei extract, avocado extract, sweet hydrangea leaf extract, Althea extract, Arnica extract, ginkgo extract, fennel extract, turmeric extract, oolong tea extract, Scutellaria root extract, phellodendron bark extract, barley extract, water cress extract, marine algae extract, hydrolyzed elastin, hydrolyzed wheat flour, hydrolyzed silk, chamomilla extract, Artemisia Capillaris extract, licorice extract, Hibiscus Sabdariffa extract, guanosine, Sasa Veitchii extract, walnut extract, Clematis extract, yeast extract, Arctium Lappa root extract, comfrey extract, cowberry extract, Bupleurum root extract, umbilical cord extract, Salvia extract, Saponaria officinalis extract, bamboo grass extract, hawthorn extract, Lentinus Edodes extract, Rehmannia Glutinosa extract, Lithospermum Officinale extract, Tilia Japonica extract, Filipendula multijuga extract, calamus root extract, Betula Alba extract, horsetail extract, Lonicera Japonica extract, Hedera Helix extract, Crataegus Oxyacantha extract, Sambucus Nigra extract, Achillea Millefolium extract, Mentha Piperita extract, Malva Sylvestris extract, Swertia Japonica extract, jujube extract, Thymus Vulgaris extract, clove extract, cogon grass extract, Citrus Unshiu peel extract, Citrus Aurantium Amara peel extract, Houttuynia Cordata extract, natto extract, carrot extract, Rosa Canina extract, hibiscus extract, Ophiopogon tuber extract, parsley extract, honey, Parietaria Officinalis extract, Isodonis Japonicus extract, bisabolol, coltsfoot extract, butterbur sprout extract, Poria Cocos extract, Ruscus Aculeatus extract, propolis, peppermint extract, linden extract, hops extract, pine extract, horse chestnut extract, Lysichiton Camtschatcensis extract, Sapindus Mukorossi extract, peach leaf extract, cornflower extract, Eucalyptus Globulus extract, Citrus Junos extract, Artemisia Princeps extract, lavender extract, lettuce extract, Astragalus Sinicus extract, rose extract, rosemary extract, Anthemis Nobilis extract, royal jelly extract or the like.

The dissolution aid is added so as not to precipitate the components contained in the adhesive mass layer. Examples of the dissolution aid can include crotamiton; N-methylpyrrolidone; polyalkylene glycol such as polyethylene glycol (PEG) or polybutylene glycol; fatty acid esters such as isopropyl myristate or diethyl adipate; oxyalkylene fatty acid esters such as polyethylene glycol monostearate; fatty acid esters such as polyoxyalkylene sorbitan fatty acid ester; polyoxyethylene hydrogenated castor oil; and surfactants such as polysorbate 80. These dissolution aids can be used singly or in combination of two or more. It is preferable that the content of the dissolution aid be 0.1 to 10% by mass on the basis of the mass of the adhesive mass layer.

The crosslinker is added to adjust the progression degree of the crosslinking reaction of a polyacrylic acid or its neutralization product, or poly(methyl acrylate/2-ethylhexyl acrylate), and by adjusting the content of the crosslinker, the follow moving of the gel patch to the skin can be adjusted. As the crosslinker, it is possible to use a crosslinker generally used for a gel patch.

There are no particular limitations to humectant as long as the humectant can suppress the evaporation of moisture from the adhesive mass layer with the elapse of time. As the humectants, polyhydric alcohols, for example, sorbitol, ethylene glycol, propylene glycol, polyethylene glycol, liquid paraffin, 1,3-propanediol, and 1,4-butanediol are mentioned. These humectants can be used singly or in combination of two or more. It is preferable that the content of the humectant be 3 to 70% by mass on the basis of the mass of the adhesive mass layer.

The cooling agents offer a cold feeling and a cool feeling to the user in the event of using the gel patch, and may have aromatic odor. Examples of the cooling agents can include thymol, 1-menthol, dl-menthol, 1-isopulegol, peppermint oil, and it is preferable to use 1-menthol. It is preferable for the content of the cooling agent to be 0.5 to 3% by mass on the basis of the mass of the adhesive mass layer.

The stabilizer improves the storage stability of the physiologically active substance to light (particularly, ultraviolet), heat or oxygen. Examples of the stabilizer include oxybenzone, dibutylhydroxytoluene (BHT), sodium edetate, UV absorbers (for example, dibenzoylmethane derivatives). It is preferable for the content of the stabilizer to be 0.01 to 1% by mass on the basis of the mass of the adhesive mass layer.

The inorganic powder is added to adjust a sticky feeling after the use of the gel patch, or suppress the seeping through the backing fabric of the adhesive mass layer. Examples of inorganic powders include alumina, light silica, titanium dioxide, synthetic aluminum silicate. It is preferable for the content of the inorganic powder to be 0.1 to 10% by mass on the basis of the mass of the adhesive mass layer.

The gel patch may comprise a release liner. The release liner is layered onto the opposite surface to the backing fabric, with being centered on the adhesive mass layer. With the release liner, there is a tendency to suppress decrease in the content of water in the adhesive mass layer during the storage, and reduce the adhesion of alien substances or the like to the adhesive mass layer.

The material of the release liner is not particularly limited, and it is possible to use a liner generally known to a person skilled in the art. Examples of the material of the release liner include polyethylene, polypropylene, polyethylene terephthalate and paper and these can be used singly or in combination of two or more.

The gel patch can be stored within a pouch. By storing the gel patch within the pouch, it is possible to suppress a decrease in the content of water in the adhesive mass layer, and it is possible to reduce the adhesion of alien substances or the like to the adhesive mass layer.

For example, it is possible to produce a gel patch of the present embodiment as follows. That is to say, glycol salicylate, a water-soluble polymer (polyacrylic acid and/or its neutralization product or the like), water and glycerin are mixed, and if necessary, the above-described other ingredients are added, to obtain an adhesive mass paste. Next, the resulting adhesive mass paste is uniformly spread over a release liner, and the backing fabric is layered onto the release liner, and then the release liner was peeled to obtain a gel patch with the adhesive mass layer formed on the backing fabric.

It is preferable for the content of glycerin to be 8 to 14 times the content of glycol salicylate on the basis of mass, and it is more preferable to be 8 to 13.6 times. Also, it is preferable to adjust the pH of the adhesive mass layer to be 4.9 to 5.1. The adhesive mass paste may contain poly(methyl acrylate/2-ethylhexyl acrylate). It is preferable that the water-soluble polymer contain a polyacrylic acid or a neutralized polyacrylate.

It is preferable for the water-soluble polymer to contain polyacrylic acid or a neutralized polyacrylate.

### Examples

Hereinbelow, the present invention is more specifically illustrated by showing Examples and Comparative Examples.

### (Preparation of the gel patch)

In accordance with the composition described in Table 1, the ingredients were sufficiently mixed to prepare an adhesive mass paste. The resulting adhesive mass paste was uniformly spread over a release liner, and a backing fabric was layered onto the release liner, and then the release liner was peeled to obtain each of the gel patches of Examples 1 to 8 as well as Comparative Examples 1 and 2.

**[Table 1]**

| | Example 1 | Example 2 | Example 3 | Example 4 | Example 5 |
|---|---|---|---|---|---|
| 1-Menthol | 1 | 1 | 1 | 1 | 1 |
| Glycol salicylate | 2 | 2 | 2 | 2 | 2 |
| Tocopherol acetate | 1 | 1 | 1 | 1 | 1 |
| Gelatin | 2.5 | 2.5 | 2.5 | 2.5 | 2.5 |
| Partially neutralized polyacrylate | 3 | 3 | 3 | 3 | 3 |
| Polyacrylic acid | 3 | 3 | 3 | 3 | 3 |
| Polyvinyl alcohol | 2.5 | 2.5 | 2.5 | 2.5 | 2.5 |
| Crosslinker | 0.05 | 0.05 | 0.05 | 0.05 | 0.05 |
| tartaric acid | 0 | 0.25 | 0.5 | 0 | 0.5 |
| Glycerin | 30 | 30 | 30 | 28 | 28 |
| Other ingredients | 2.52 | 2.52 | 2.52 | 2.52 | 2.52 |
| Purified water | 52.43 | 52.18 | 51.93 | 54.43 | 53.93 |
| Total | 100 | 100 | 100 | 100 | 100 |

| | Example 6 | Example 7 | Example 8 | Comp. Example 1 | Comp. Example 2 |
|---|---|---|---|---|---|
| 1-Menthol | 1 | 1 | 1 | 1 | 1 |
| Glycol salicylate | 2 | 2 | 2 | 2 | 2 |
| Tocopherol acetate | 1 | 1 | 1 | 1 | 1 |
| Gelatin | 2.5 | 2.5 | 2.5 | 2.5 | 2.5 |
| Partially neutralized polyacrylate | 3 | 3 | 3 | 3 | 3 |
| Polyacrylic acid | 3 | 4 | 3 | 3 | 3 |
| Polyvinyl alcohol | 2.5 | 2.5 | 2.5 | 2.5 | 2.5 |
| Crosslinker | 0.05 | 0.05 | 0.05 | 0.05 | 0.05 |
| tartaric acid | 0 | 0 | 0 | 0 | 0 |
| Glycerin | 23 | 23 | 16 | 35 | 10 |
| Other ingredients | 2.52 | 2.52 | 2.52 | 2.52 | 2.52 |
| Purified water | 59.43 | 58.43 | 66.43 | 47.43 | 72.43 |
| Total | 100 | 100 | 100 | 100 | 100 |

### (Evaluation)

By referring to the description of the Japanese Pharmacopoeia, the pH of the adhesive mass layers of the resulting gel patches was measured, and the measured values are shown in Table 2. Also, after the storage of each gel patch at 60°C for one month the content of glycol salicylate contained in the adhesive mass layer was measured by gas chromatography. The contents of glycol salicylate in the gel patch after the storage with respect to the content of glycol salicylate (initial value) in the gel patch at the time of preparation are shown in Table 2.

Comparing the gel patches of Examples 1 to 3, even among the gel patches with the identical content of glycerin, a higher content of tartaric acid led to a lower pH of the adhesive mass layer and a higher storage stability of glycol salicylate. Also, comparing the gel patches of Examples 1, 4, 6, and 8, as the content of glycerin decreased, storage stability of glycol salicylate was improved. On the other hand, for the gel patch of Comparative Example 1 with a glycerin content in the adhesive mass layer of 35% by mass, the storage stability of glycol salicylate decreased. Also, the gel patch of Comparative Example 2 with a glycerin content in the adhesive mass layer of 10% by mass had a higher storage stability of glycol salicylate, but the moisture retention was not sufficient as a gel patch.

**[Table 2]**

| | Example 1 | Example 2 | Example 3 | Example 4 | Example 5 |
|---|---|---|---|---|---|
| pH of adhesive mass | 5.1 | 4.9 | 4.7 | 5.1 | 4.7 |
| Content of glycol salicylate (% based on initial value) | 89.1 | 91.2 | 92.6 | 89.5 | 92.8 |

| | Example 6 | Example 7 | Example 8 | Comp. Example 1 | Comp. Example 2 |
|---|---|---|---|---|---|
| pH of adhesive mass | 5.1 | 4.9 | 5.1 | 5.1 | 5.1 |
| Content of glycol salicylate (% based on initial value) | 90.1 | 92.2 | 91.8 | 88 | 93 |

### (Preparation of the gel patch)

In accordance with the composition described in Table 3, the ingredients were sufficiently mixed to prepare an adhesive mass paste. The resulting adhesive mass paste was uniformly spread over a release liner, and a backing fabric was layered onto the release liner, followed by the peeling of the release liner to obtain each of the gel patches of Examples 9 to 12.

**[Table 3]**

| | Example 9 | Example 10 | Example 11 | Example 12 |
|---|---|---|---|---|
| 1-Menthol | 1 | 1 | 1 | 1 |
| Glycol salicylate | 2 | 2 | 2 | 2 |
| Tocopherol acetate | 1 | 1 | 1 | 1 |
| Gelatin | 2.5 | 2.5 | 2.5 | 2.5 |
| Partially neutralized polyacrylate | 3 | 3 | 3 | 3 |
| Polyacrylic acid | 3 | 3 | 3 | 3 |
| Polyvinyl alcohol | 2.5 | 2.5 | 2.5 | 2.5 |
| NIKASOL | 8.35 | 8.35 | 8.35 | 8.35 |
| Crosslinker | 0.05 | 0.05 | 0.05 | 0.05 |
| Glycerin | 28 | 28 | 23 | 23 |
| D-sorbitol | 0 | 7 | 0 | 7 |
| Other ingredients | 2.52 | 2.52 | 2.52 | 2.52 |
| Purified water | 46.08 | 39.08 | 51.08 | 44.08 |
| Total | 100 | 100 | 100 | 100 |

### (Evaluation)

By referring to the description of the Japanese Pharmacopoeia, the pH of the adhesive mass layers of the resulting gel patches was measured, and the measured values are shown in Table 4. Also, after the storage of each gel patch at 60°C for one month, the content of glycol salicylate in the adhesive mass layer was measured by gas chromatography. The contents of glycol salicylate in the gel patch after the storage with respect to the content of glycol salicylate in the gel patch at the time of preparation are shown in Table 4.

**[Table 4]**

| | Example 9 | Example 10 | Example 11 | Example 12 |
|---|---|---|---|---|
| pH of adhesive mass | 5.1 | 5.1 | 5.0 | 4.9 |
| Content of glycol salicylate (% based on initial value) | 89.5 | 90.0 | 92.0 | 92.3 |

The gel patch of Example 11 had a lower content of glycerin than that of the gel patch of Example 9, and the stability of glycol salicylate was further improved. This tendency is also found for the gel patches of Examples 10 and 12.

## Claims

1. A gel patch comprising an adhesive mass layer on a backing fabric, wherein
the adhesive mass layer comprises glycol salicylate, a water-soluble polymer, water and glycerin and poly(methyl acrylate/2-ethylhexyl acrylate), and
a content of glycerin is 8 to 15 times a content of glycol salicylate on the basis of mass, wherein a pH of the adhesive mass layer is 4.7 to 5.1.

2. The gel patch according to claim 1, wherein the content of glycerin is 8 to 14 times the content of glycol salicylate on the basis of mass.

3. The gel patch according to any one of claims 1 to 2, wherein the water-soluble polymer comprises a polyacrylic acid or a neutralized polyacrylate.

## Patentansprüche

1. Gelpflaster, umfassend eine Klebemassenschicht auf einem Trägergewebe, wobei
die Klebemassenschicht Glykolsalicylat, ein wasserlösliches Polymer, Wasser und Glycerin und Poly(methylacrylat/2-ethylhexylacrylat) umfasst, und
ein Gehalt an Glycerin das 8- bis 15-fache eines Gehalts an Glykolsalicylat auf der Basis der Masse beträgt,
wobei der pH-Wert der Klebemassenschicht 4,7 bis 5,1 beträgt.

2. Gelpflaster nach Anspruch 1, wobei der Gehalt an Glycerin das 8- bis 14-fache des Gehalts an Glykolsalicylat auf der Basis der Masse beträgt.

3. Gelpflaster nach einem der Ansprüche 1 bis 2, wobei das wasserlösliche Polymer eine Polyacrylsäure oder ein neutralisiertes Polyacrylat umfasst.

## Revendications

1. Timbre de gel comprenant une couche de masse adhésive sur un textile dorsal,
la couche de masse adhésive comprenant du salicylate de glycol, un polymère soluble dans l'eau, de l'eau et de la glycérine et du poly(acrylate de méthyle/acrylate de 2-éthylhexyle), et
une teneur de la glycérine étant de 8 à 15 fois une teneur du salicylate de glycol sur la base de la masse, un pH de la couche de masse adhésive étant de 4,7 à 5,1.

2. Timbre de gel selon la revendication 1, la teneur de la glycérine étant de 8 à 14 fois la teneur du salicylate de glycol sur la base de la masse.

3. Timbre de gel selon l'une quelconque des revendications 1 à 2, le polymère soluble dans l'eau comprenant un poly(acide acrylique) ou un polyacrylate neutralisé.
